# EUROPÄISCHE PATENTSCHRIFT

(11) **EP 1 382 342 B1**
(45) Veröffentlichungstag und Bekanntmachung des Hinweises auf die Patenterteilung: **20.09.2006**
(21) Anmeldenummer: 02015982.8
(22) Anmeldetag: 18.07.2002
(51) Int. Cl.: A61K 31/704, A61K 31/60, A61P 17/10, A61K 9/16, A61K 9/127, A61K 36/484

(54) **Verwendung von Salicylsäure und Glyzyrrhetinsäure enhaltende Zusammensetzungen zur Aknebehandlung**
Use of compositions comprising glycyrrhetinic acid and salicylic acid for acne treatment
Utilisation de compositions comprenant de l'acide glycyrrhetinique et l'acide salicylique pour le traitement de l'acné

(43) Veröffentlichungstag der Anmeldung: 21.01.2004
(73) Patentinhaber: Cognis IP Management GmbH, 40589 Düsseldorf (DE)
(72) Erfinder: Arias, Carmen, 08190 Sant Cugat de Vallés (ES); Buchwald-Werner, Sybille, Dr., 40589 Düsseldorf (DE); Fabry, Bernd, Dr., 41352 Korschenbroich (DE); Rull Prous, Santiago, 08034 Barcelona (ES)

(56) Entgegenhaltungen:
- WO-A-01/01926
- WO-A-01/01927
- WO-A-01/01928
- WO-A-01/01929
- WO-A-93/21899
- WO-A-02/076479
- ES-A- 2 137 125
- FR-A- 2 750 050
- DATABASE WPI Section Ch, Week 199828 Derwent Publications Ltd., London, GB; Class B05, AN 1998-316678 XP002220283 & JP 10 114648 A (KAO CORP), 6. Mai 1998 (1998-05-06)
- PATENT ABSTRACTS OF JAPAN vol. 016, no. 207 (C-0941), 18. Mai 1992 (1992-05-18) & JP 04 036215 A (SUNSTAR INC), 6. Februar 1992 (1992-02-06)
- DATABASE WPI Section Ch, Week 199338 Derwent Publications Ltd., London, GB; Class A96, AN 1993-297779 XP002220317 & JP 05 208907 A (MIKASA SEIYAKU KK), 20. August 1993 (1993-08-20)
- DATABASE WPI Section Ch, Week 199930 Derwent Publications Ltd., London, GB; Class A96, AN 1999-352793 XP002220315 & JP 11 130680 A (HASEGAWA CO LTD), 18. Mai 1999 (1999-05-18)
- DATABASE WPI Section Ch, Week 199805 Derwent Publications Ltd., London, GB; Class B04, AN 1998-045450 XP002220316 & JP 09 291012 A (SUNSTAR CHEM IND CO LTD) , 11. November 1997 (1997-11-11)

## Beschreibung

### Gebiet der Erfindung

Die Erfindung befindet sich auf dem Gebiet der botanischen Extrakte und betrifft die Verwendung einer Wirkstoffmischung als Anti-Aknemittel, enthaltend Salicylsäure und deren Derivate zusammen mit den Wirkstoffen der *Glycyrrhiza glabra,* Mikrokapseln und Pro-Liposomen, die diese Mischungen enthalten sowie Verfahren zur deren Herstellung.

### Stand der Technik

Unter dem Begriff Akne versteht der Fachmann eine Hauterkrankung, die sich durch entzündliche und nicht entzündliche Knötchen auszeichnen und die zur Bildung von Pusteln, Abszessen und schließlich Narben führen kann. Obschon die Ursachen unterschiedlich sind, lässt sich Akne letztlich auf verstopfte Haarfollikel (Komedone) zurückführen. Neben einer hormonell bedingten Verstopfung der Haarfollikel-Mündungen durch Körperfette, besteht eine wesentliche Ursache für die Entstehung von Akne in der Entwicklung gewebeschädigender freier Fettsäuren und Enzyme durch Bakterien, wie beispielsweise Propionibacterium acnes.

Aus dem Stand der Technik sind eine ganze Reihe von Wirkstoffen bekannt, die zur Bekämpfung von Akne mit mehr oder minder großem Erfolg eingesetzt werden können. So wird beispielsweise in der Spanischen Patentschrift **ES 9702410 B1** (Vinyals) die Verwendung des Zinksalzes der Glyzyrrhetinsäure gegen Akne vorgeschlagen. Als weitere Wirkstoffe, die im wesentlichen gegen eine überhöhte Sebumproduktion wirksam sind, seien beispielsweise Benzoylperoxid, Hexaclorophen, Dodecylbenzolsulfonsäure, 2,2',2'-Nitrilotriethanol-Salze, Dexpanthenol oder Resorcin genannt. Keiner dieser Stoffe zeigt jedoch für sich alleine genommen eine befriedigende Wirkung gegenüber den Keimen, die maßgeblich am Entstehen von Akne, speziell von Acne vulgaris, beteiligt sind.

Die Aufgabe der Erfindung hat daher darin bestanden, neue Wirkstoffkombinationen zur Verfügung zu stellen, die einerseits gegenüber den am Entstehen von Akne beteiligten Bakterien eine verbesserte Wirkung aufweisen und zudem den Heilungsprozess vorzugsweise durch anti-inflammatorische Eigenschaften unterstützen.

### Beschreibung der Erfindung

Gegenstand der Erfindung ist die Verwendung einer Wirkstoffmischung, enthaltend
(a) Salicylsäure sowie deren Derivate und
(b) Glyzyrrhetinsäure sowie deren Derivate
zur Herstellung eines Medikamentes zur Bekämpfung von Akne.

Überraschenderweise wurde gefunden, dass Salicylsäure und Glyzyrrhetinsäure sowie deren Derivate, die jeweils für sich als Wirkstoffe zur Bekämpfung von Akne bekannt sind, eine synergistische Leistungsverstärkung zeigen. Besonders effizient hat sich erwiesen, die Wirkstoffe in Mikrokapseln oder Pro-Liposomen einzuschließen, aus denen sie gezielt freigesetzt werden können. Dabei haben sich Mikrokapseln auf Basis von Chitosan wiederum als besonders vorteilhaft erwiesen, da diese kationischen Biopolymeren selbst über eine anti-inflammatorische Wirkung verfügen und damit den Heilungsprozess unterstützen.

### Salicylsäure und deren Derivate

Salicylsäure (Komponente a) stellt eine aromatische Carbonsäure dar. Im Sinne der Erfindung ist es aber nicht nur möglich, dass die reine Säure zum Einsatz gelangt, alternativ oder in Abmischung kommen auch die Alkali-, Erdalkali-, Ammonium-, Alkylammonium-, Alkanolammonium-, Glucammonium- und Zinksalzen der Salicylsäure, die Ester der Salicylsäure mit linearen oder verzweigten aliphatischen Alkoholen mit 1 bis 18 Kohlenstoffatomen sowie die Voll- oder Partialester der Salicylsäure mit Polyolen mit 2 bis 15 Kohlenstoffatomen und mindestens 2 Hydroxylgruppen in Frage.

Typische Beispiele sind die Natrium-, Kalium-, Ammonium-, Triethanolammonium-, Glucammonium und Zinksalze der Salicylsäure, die Ester der Salicylsäure mit Methanol, Ethanol, den isomeren Propanolen und Butanolen sowie Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol und deren Gemischen. Weitere Beispiele sind die Voll- oder Partialester der Salicylsäure mit Glycerin; Alkylenglycolen, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technischen Oligoglyceringemischen mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucosiden, insbesondere solche mit 1 bis 8 Kohlenstoffen im Alkylrest, wie beispielsweise Methyl- und Butylglucosid; Zuckeralkoholen mit 5 bis 12 Kohlenstoffatomen, wie beispielsweise Sorbit oder Mannit; Zuckern mit 5 bis 12 Kohlenstoffatomen, wie beispielsweise Glucose oder Saccharose; Aminozuckern, wie beispielsweise Glucamin; oder Dialkoholaminen, wie Diethanolamin oder 2-Amino-1,3-propandiol.Vorzugsweise setzt man als Komponente (a) Azelainsäure oder deren Zinksalz ein.

### Glyzyrrhetinsäure und deren Derivate

Glyzyrrhetinsäure (Komponente b, s. Abbildung) und deren Derivate finden sich als Komponenten in Extrakten der *Glycyrrhiza glabra;* Derivate mit einer 1,2-β-Zuckerbindung sind für den Lakritzgeschmack verantwortlich.

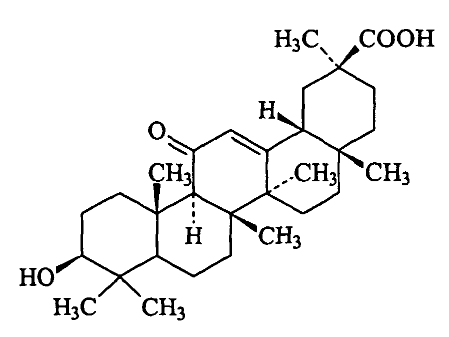

Im Sinne der Erfindung ist es aber nicht nur möglich, dass die reine Säure zum Einsatz gelangt, alternativ oder in Abmischung kommen auch die Alkali-, Erdalkali-, Ammonium-, Alkylammonium-, Alkanolammonium-, Glucammonium- und Zinksalzen der Glyzyrrhetinsäure, die Ester der Glyzyrrhetinsäure mit linearen oder verzweigten aliphatischen Alkoholen mit 1 bis 18 Kohlenstoffatomen sowie die Voll- oder Partialester der Glyzyrrhetinsäure mit Polyolen mit 2 bis 15 Kohlenstoffatomen und mindestens 2 Hydroxylgruppen in Frage.

Typische Beispiele sind die Natrium-, Kalium-, Ammonium-, Triethanolammonium-, Glucammonium und Zinksalze der Glyzyrrhetinsäure, die Ester der Glyzyrrhetinsäure mit Methanol, Ethanol, den isomeren Propanolen und Butanolen sowie Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol und deren Gemischen. Weitere Beispiele sind die Voll- oder Partialester der Glyzyrrhetinsäure mit Glycerin; Alkylenglycolen, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technischen Oligoglyceringemischen mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucosiden, insbesondere solche mit 1 bis 8 Kohlenstoffen im Alkylrest, wie beispielsweise Methyl- und Butylglucosid; Zuckeralkoholen mit 5 bis 12 Kohlenstoffatomen, wie beispielsweise Sorbit oder Mannit; Zuckern mit 5 bis 12 Kohlenstoffatomen, wie beispielsweise Glucose oder Saccharose; Aminozuckern, wie beispielsweise Glucamin; oder Dialkoholaminen, wie Diethanolamin oder 2-Amino-1,3-propandiol. Vorzugsweise setzt man als Komponente (b) das Kalium-, Ammonium- und/oder Zinksalz der Glyzyrrhetinsäure oder deren Ester mit Fettalkoholen mit 16 bis 18 Kohlenstoffatomen ein.

Anstelle der reinen Glyzyrrhetinsäure bzw. deren Derivate kann in einer bevorzugten Ausführungsform der vorliegenden Erfindung der Extrakt der *Glycyrrhiza glabra* direkt eingesetzt werden, welcher üblicherweise eine Mischung von 18-β-Glyzyrrhetinsäure, 18-β-Glyzyrrhetinsäure-Kaliumsalz und 18-β-Glyzyrrhetinsäurestearat darstellt. Glyzyrrhetinsäure selbst ist in einer Reinheit von 98 Gew.-% beispielsweise unter der Marke Plantactiv® GLA 18 (Cognis Iberia, S.L.) im Handel erhältlich.

Die Komponenten (a), (b) und gegebenenfalls (c) können in den Mikrokapseln in Mengen von zusammen 1 bis 30 Gew.-% - bezogen auf das Kapselgewicht - vorhanden sein.

### Anti-Aknemittel

Die Endzubereitungen können die Komponenten (a) und (b) im Gewichtsverhältnis 90 : 10 bis 10 : 90 enthalten, wobei beträchtliche Synergien im Bereich von 75 : 25 bis 25 : 75 und insbesondere 60 : 40 bis 40 : 60 festgestellt werden. Als weitere Komponenten können die erfindungsgemäß zu verwendenden Zubereitung andere gegen Akne wirksame oder anti-inflammatorische Stoffe enthalten, wie beispielsweise Pflanzenextrakte oder Vitamin B6. Typische Beispiele für geeignete Pflanzenextrakte sind die Wirkstoffe folgender Pflanzen : *Aesculus hippocastanum* (Rosskastanie), *Argania spinosa, Babtista tinctoria* (wilder Indigo), *Cantella asiatica, Camelilla sinensis* (Grüner Tee), *Chamonella recutita* (Kamille), *Ginkgo biloba* (Ginkgo), *Oleo europea* (Olive), *Litschi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna acontifolia,* und *Vitis vinifera* (wilder Wein).

In einer bevorzugten Ausführungsform der Erfindung gelangen solche Zubereitungen zum Einsatz, die - bezogen auf die Wirkstoffe -
(a) 10 bis 90, vorzugsweise 20 bis 80 Gew.-% Salicylsäure und/oder deren Derivate,
(b) 10 bis 90, vorzugsweise 20 bis 80 Gew.-% Glyzyrrhetinsäure und/oder deren Derivate, und
(c) 0 bis 20, vorzugsweise 5 bis 10 Gew.-% Pflanzenextrakte und/oder Vitamin B6
mit der Maßgabe enthalten, dass sich die Mengen zu 100 Gew.-% ergänzen.

### Mikrokapseln und Pro-Liposomen

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].**

### Pro-Liposomen

Anstelle der beschriebenen Mikrokapseln kommen als Träger für die Wirkstoffgemische auch Pro-Liposomen in Frage. Zur Begriffsklärung sei darauf hingewiesen, dass die Pro-Liposomen kein Wasser enthalten und dieses erst dann unter Bildung von echten Liposomen aufnehmen, wenn sie in eine wässrige Umgebung eingebracht werden. Ein weiterer Gegenstand der Erfindung betrifft daher pro-liposomale Wirkstoffgemische, die die Komponenten (a) und (b) aufweisen, und welche man erhält, indem man die Gemische in pharmazeutisch bzw. kosmetisch akzeptablen Lösemitteln mit Lecithinen und/oder Phospholipiden behandelt.

Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet.

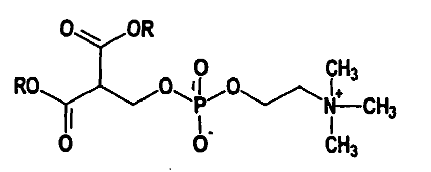

In der obigen Formel ist ein Lecithin schematisch angegeben, wobei R typischerweise für lineare aliphatische Kohlenwasserstoffreste mit 15 bis 17 Kohlenstoffatomen und bis zu 4 cis-Doppelbindungen steht. Als Beispiele für natürliche Lecithine, die zur Verkapselung in Frage kommen, seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch zur liposomalen Verkapselung Sphingosine bzw. Sphingolipide in Frage.

### Herstellverfahren Pro-Liposomen

Pro-Liposomen können im Sinne der Erfindung erhalten werden, indem man die Wirkstoffe in pharmazeutisch bzw. kosmetisch akzeptablen Lösemitteln mit Lecithinen und/oder Phospholipiden behandelt. Hierzu werden die Wirkstoffe üblicherweise entweder in einem Lösungsmittel vorgelegt und mit den Lecithinen bzw. Phospholipiden bei Temperaturen im Bereich von 30 bis 70 °C in Kontakt gebracht oder aber die wasserfreien Gemische werden in eine Lösung der Lecithine bzw. Phospholipide eingerührt. Die Wirkstoffe und die Lecithine und/oder Phospholipide können dabei im Gewichtsverhältnis 1 : 20 bis 5 : 1, vorzugsweise 1 : 2 bis 4 : 1 eingesetzt werden. Als Lösemittel eignen sich vorzugsweise niedere Alkohole mit 1 bis 4 Kohlenstoffatome, wie z.B. Ethanol oder Polyole, welche in der Regel 2 bis 15 Kohlenstoffatome und mindestens zwei Hydroxylgruppen besitzen; hier ist Propylenglycol bevorzugt.

### Beispiele

### Herstellbeispiele

**Beispiel 1.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis Deutschland GmbH & Co. KG), 4 g Salicylsäure, 4 g Glyzyrrhetinsäure-Zinksalz, Phenonip® (Konservierungsmittelmischung enthaltend Phenoxyethanol und Parabene) und 0,5 g Polysorbat-20 in ad 100 g Wasser versetzt. Die erhaltene Matrix wurde filtriert, auf 60 °C erwärmt und in eine 0,5 Gew.-%ige Natriumalginatlösung getropft. Nach dem Sieben wurde eine wässrige Zubereitung erhalten, die 8 Gew.-% Mikrokapseln mit einem mittleren Durchmesser von 1 mm enthielt.

**Beispiel 2.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 4 g Salicylsäure-Kaliumsalz, 4 g Glyzyrrhetinsäure-Zinksalz, 0,5 g Phenonip® und 0,5 g Polysorbat-20 in ad 100 g Wasser versetzt. Die erhaltene Matrix wurde filtriert, auf 50°C temperiert und unter starken Rühren im 2,5fachen Volumen Paraffinöl, das zuvor auf 15 °C gekühlt worden war, dispergiert. Die Dispersion wurde anschließend mit einer wässrigen Lösung enthaltend 1 Gew.-% Natriumlaurylsulfat und 0,5 Gew.-% Natriumalginat und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 3**. In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 4 g Salicylsäure-Zinksalz, 4 g Glyzyrrhetinsäure-Zinksalz, 0,5 g Phenonip® und 0,5 g Polysorbat-20 in ad 100 g Wasser versetzt. Die erhaltene Matrix wurde filtriert, auf 60 °C erwärmt und in eine 15 Gew.-%ige Lösung von Sodium Laureth Sulfate getropft. Nach dem Sieben wurde eine wässrige Zubereitung erhalten, die 9 Gew.-% Mikrokapseln mit einem mittleren Durchmesser von 1 mm enthielt.

**Beispiel 4.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan , 4 g Salicylsäure, 4 g *Glycyrrhiza glabra*-Extrakt, 0,5 g Phenonip® und 0,5 g Polysorbat-20 in ad 100 g Wasser versetzt. Die erhaltene Matrix wurde filtriert, auf 60 °C erwärmt und in eine 15 Gew.-%ige Lösung von Natriumpyrophosphat getropft. Nach dem Sieben wurde eine wässrige Zubereitung erhalten, die 8 Gew.-% Mikrokapseln mit einem mittleren Durchmesser von 1 mm enthielt.

**Beispiel 5.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 4 g Salicylsäure-Zinksalz, 4 g *Glycyrrhiza glabra-*Extrakt*,* 0,5 g Phenonip® und 0,5 g Polysorbat-20 in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Natriumpyrophosphatlösung und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 10 Gew.-% Mikrokapseln mit einem mittleren Durchmesser von 1 mm enthielt.

**Beispiel 6.** In einem 500-ml-Dreihalskolben mit Rührer und Rückflusskühler wurden in der Siedehitze 3 g Gelatine in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 4 g Salicylsäure-Zinksalz, 4 g *Glycyrrhiza glabra-*Extrakt, 0,5 g Phenonip® in ad 100 g Wasser versetzt. Die erhaltene Matrix wurde filtriert, auf 60 °C erwärmt und in eine 0,5 Gew.-%ige Lösung von Hydagen® SCD (succinyliertes Chitosan, Cognis Deutschland GmbH & Co. KG)) getropft. Nach dem Sieben wurde eine wässrige Zubereitung erhalten, die 8 Gew.-% Mikrokapseln mit einem mittleren Durchmesser von 1 mm enthielt.

**Beispiel 7.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 3 g Salicylsäure, 3 g *Glycyrrhiza glabra-*Extrakt, 2 g Camellia sinensis-Extrakt (Herbalia® Green Tea, Cognis Iberia, S.L.), 0,5 g Phenonip® und 0,5 g Polysorbat-20 in ad 100 g Wasser versetzt. Die erhaltene Matrix wurde filtriert, auf 60 °C erwärmt und in eine 0,5 Gew.-%ige Natriumalginatlösung getropft. Zum Erhalt von Mikrokapseln gleichen Durchmessers wurden die Zubereitungen anschließend gesiebt.

**Beispiel 8.** In einer Rührapparatur wurden 0,5 g Konservierungsmittel (Phenonip®) in 50 g einer 2 Gew.-%igen wässrigen Zubereitung von Carboxymethylcellulose gelöst und die Mischung auf pH = 3,5 eingestellt. Anschließend wurde unter starkem Rühren eine Mischung bestehend aus 5 g Salicylsäure, 5 g Glyzyrrhetinsäure-Zinksalz und Sorbitanmonostearat+20EO hinzugegeben. Danach wurde unter weiterem Rühren eine solche Menge einer 1 Gew.-%igen Lösung von Chitosan in Glycolsäure hinzugegeben, dass sich eine Chitosankonzentration von 0,075 Gew.-% - bezogen auf die Zubereitung - einstellte. Anschließend wurde der pH-Wert durch Zugabe von Triethanolamin auf 5,5 angehoben und die entstandenen Mikrokapseln dekantiert.

**Beispiel 9.** In einer Rührapparatur wurden 0,5 g Konservierungsmittel (Phenonip®) in 50 g einer 2 Gew.-%igen wässrigen Zubereitung von Polyacrylsäure (Pemulen® TR-2) gelöst, wobei sich ein pH-Wert von 3 einstellte. Anschließend wurde unter starkem Rühren eine Mischung bestehend aus 5 g Salicylsäure, 5 g *Glycyrrhiza glabra*-Extrakt, und Sorbitanmonolaurat+15EO hinzugegeben. Danach wurde unter weiterem Rühren eine solche Menge einer 1 Gew.-%igen Lösung von Chitosan in Glycolsäure hinzugegeben, dass sich eine Chitosankonzentration von 0,01 Gew.-% - bezogen auf die Zubereitung - einstellte. Anschließend wurde der pH-Wert durch Zugabe von Triethanolamin auf 5,5 angehoben und die entstandenen Mikrokapseln dekantiert.

**Beispiel 10.** In einer Rührapparatur wurden 5 g Salicylsäure und 5 g Glyzyrrhetinsäure-Zinksalz vorgelegt und mit 100 g einer 20 Gew.-%igen Lösung von Sojalecithin in Propylenglycol versetzt. Die Mischung wurde auf 65 °C erwärmt und solange gerührt, bis eine homogene, klare Lösung resultierte.

**Beispiel 11.** In einer Rührapparatur wurden 5 g Salicylsäure und 5 g eines gefriergetrockneten. Extraktes von *Glycyrrhiza glabra* vorgelegt und mit 100 g einer 20 Gew.-%igen Lösung von Sojalecithin in Propylenglycol versetzt. Die Mischung wurde auf 65 °C erwärmt und solange gerührt, bis eine homogene, klare Lösung resultierte.

### Antibakterielle Wirksamkeit

Im folgenden wurden folgende Wirkstoffe bzw. deren Gemische getestet:
- A1: Salicylsäure
- A1: Salicylsäure-Kaliumsalz
- A2: Salicylsäure-Zinksalz
- B1: Glyzyrrhetinsäure-Zinksalz
- B2: Extrakt der *Glycyrrhiza glabra*

Die antibakterielle Wirksamkeit der Wirkstoffe und Wirkstoffmischungen wurde mit Hilfe der Diffusionsmethode auf Agar-Platten bzw. der Agar-Verdünnungsmethode untersucht. Dabei wird zunächst ein rundes Filterpapier definierten Durchmessers mit der Testlösung getränkt und dieses dann auf die Oberfläche einer Agarplatte aufgebracht, welche zuvor mit den Testmikroorganismen inokuliert worden war. Anschließend wird nach definierten Zeiten die Größe der Inhibierungszonen bestimmt. Diese Technik erlaubt es insbesondere die MIC (Minimum Inhibitory Concentration) als die niedrigste Testsubstanzkonzentration zu bestimmen, mit der noch eine vollständige Inhibierung der Mikroorganismen erzielt werden kann.

*Agar-Diffusionsmethode.* Das Inokulum wurde unter Verwendung einer frischen Kultur in einer stationären Wachstumsphase (nach ca. 18-24 h) in einer BHI-Lösung (Brain-Heart-InfusionI) hergestellt. Die Bakteriensuspension wurde auf 0,5 MacFarland-Einheiten eingestellt, entsprechend 1,5 10⁸ Kolonienbildende Einheiten (cfu)/ml; anschließend wurde die Suspension mit einer Kochsalzlösung 1/100 verdünnt, um einen Wert von 1,5 10⁶ cfu/ml einzustellen. Anschließend wurde Mueller-Hinton Agar (*Staphylococcus epidermis, Staphylococcus aureus*) und Wilkins-Chalgrens Agar mit 5 Gew.-% Schafsblut (*Propionobakterium acnes*) 15 min bei 121 °C sterilisiert und dann in Petrischalen gegeben. Die Petrischalen wurden mit 2 bis 4 ml der Bakteriensuspensionen versetzt und bei Raumtemperatur getrocknet. Die Filterpapiere wurden mit 20 µl der Testsubstanzen getränkt und auf die Oberfläche der Agarplatten aufgebracht. Die inokulierten Petrischalen wurden 18 bis 24 bei 37 °C inkubiert (die Petrischalen mit Propionibacterium acnes unter anaeroben Bedingungen) und die antimikrobielle Wirksamkeit anschließend durch Bestimmung der Wachstumsinhibierungszone ermittelt. Die Ergebnisse sind in Tabelle 1 zusammengefasst. Angegeben sind ist jeweils der Durchmesser der Inhibierungsflächen in mm.

*Agar Verdünnungsmethode (MIC-Bestimmung).* Wie oben beschrieben wurden verschiedene Agars vorbereitet, mit unterschiedlichen Konzentrationen an Testsubstanzen versetzt, homogenisiert und dann getrocknet. Anschließend erfolgte die Inokulation der Petrischalen mit jeweils 2 µl der Bakteriensuspensionen. Nach dem erneuten Trocknen wurden die Petrischalen bei 37 °C 18 bis 24 h inkubiert. In Tabelle 2 sind die MIC in mg/ml angegeben, d.h. die geringsten Konzentrationen, mit denen noch eine vollständige Inhibierung des Bakterienwachstums erreicht werden kann. Es handelt sich jeweils um den Mittelwert von Doppelbestimmungen.

**Tabelle 1**

| **Inhibierungszonen [mm]** | | | |
|---|---|---|---|
| **Wirkstoffverhältnis** | **Staphylococcus aureus** | **Staphylococcus** | **Propionibacterium epidermidis acnes** |
| A1 (Blindprobe) | 5 | 4 | 6 |
| B1 (Blindprobe) | 5 | 5 | 4 |
| A1 : B1 = 75 : 25 | 11 | 12 | 11 |
| A1 : B1 = 50: 50 | 13 | 15 | 11 |
| A1 : B1 = 25 : 75 | 10 | 16 | 11 |
| A2 : B1 = 50 : 50 | 11 | 15 | 10 |
| A3 : B1 = 50 : 50 | 11 | 14 | 12 |
| A1 : B2 = 50 : 50 | 12 | 15 | 12 |

**Tabelle 2:**

| **MIC [mg/ml]** | | | |
|---|---|---|---|
| **Wirkstoffe** | **Staphylococcus epidermidis** | **Staphylococcus aureus** | **Propionibacterium acnes** |
| A1 (Blindprobe) | 1,25 | >10 | 1,25 |
| B1 (Blindprobe) | 1,25 | >10 | 1,25 |
| A1 : B1 = 75 : 25 | 1,25 | 1,25 | 0,625 |
| A1 : B1 = 50 : 50 | 0,625 | 1,25 | 0,625 |
| A1 : B1 = 25 : 75 | 1,25 | 1,25 | 0,625 |
| A2 : B1 = 50 : 50 | 1,25 | 1,25 | 0,625 |
| A3 : B1 = 50 : 50 | 0,625 | 1,25 | 0,625 |
| A1 : B2 = 50 : 50 | 0,625 | 1,25 | 0,625 |

Die Ergebnisse zeigen, dass die Wirkstoffgemische gegenüber den einzelnen Komponenten über synergistisch verbesserte antimikrobielle Eigenschaften speziell gegenüber solchen Keimen verfügen, die in die Entstehung von Akne involviert sind.

## Patentansprüche

1. Verwendung einer Wirkstoffmischung, enthaltend
(a) Salicylsäure sowie deren Derivate und
(b) Glyzyrrhetinsäure sowie deren Derivate
zur Herstellung eines Medikamentes zur Bekämpfung von Akne.

2. Verwendung nach Anspruch 1, **dadurch gekennzeichnet, dass** man als Komponente (a) Derivate der Salicylsäure einsetzt, die ausgewählt sind aus der Gruppe, die gebildet wird von Alkali-, Erdalkali-, Ammonium-, Alkylammonium-, Alkanolammonium-, Glucammonium- und Zinksalzen der Salicylsäure, den Estern der Azelainsäure mit linearen oder verzweigten aliphatischen Alkoholen mit 1 bis 18 Kohlenstoffatomen sowie den Voll- oder Partialestern der Salicylsäure mit Polyolen mit 2 bis 15 Kohlenstoffatomen und mindestens 2 Hydroxylgruppen.

3. Verwendung nach den Ansprüchen 1 und/oder 2, **dadurch gekennzeichnet, dass** man als Komponente (a) Salicylsäure oder deren Zinksalz einsetzt.

4. Verwendung nach mindestens einem der Ansprüche 1 bis 3, **dadurch gekennzeichnet, dass** man als Komponente (b) Derivate der Glyzyrrhetinsäure einsetzt, die ausgewählt sind aus der Gruppe, die gebildet wird von Alkali-, Erdalkali-, Ammonium-, Alkylammonium-, Alkanolammonium-, Glucammonium- und Zinksalzen der Glyzyrrhetinsäure, den Estern der Glyzyrrhetinsäure mit linearen oder verzweigten aliphatischen Alkoholen mit 1 bis 18 Kohlenstoffatomen sowie den Voll- oder Partialestern der Glyzyrrhetinsäure mit Polyolen mit 2 bis 15 Kohlenstoffatomen und mindestens 2 Hydroxylgruppen.

5. Verwendung nach mindestens einem der Ansprüche 1 bis 4, **dadurch gekennzeichnet, dass** man als Komponente (b) einen Extrakt der *Glycyrrhiza glabra* einsetzt.

6. Verwendung nach mindestens einem der Ansprüche 1 bis 5, **dadurch gekennzeichnet, dass** man als Komponente (b) das Kalium-, Ammonium- und/oder Zinksalz der Glyzyrrhetinsäure oder deren Ester mit Fettalkoholen mit 16 bis 18 Kohlenstoffatomen einsetzt.

7. Verwendung nach mindestens einem der Ansprüche 1 bis 6, **dadurch gekennzeichnet, dass** man die Komponenten (a) und (b) im Gewichtsverhältnis 90 : 10 bis 10 : 90 einsetzt.

8. Verwendung nach mindestens einem der Ansprüche 1 bis 7, **dadurch gekennzeichnet, dass** man als weitere Komponente (c) Pflanzenextrakte und/oder Vitamin B6 einsetzt.

9. Verwendung nach Anspruch 8, **dadurch gekennzeichnet, dass** man als Komponente (c) Extrakte von Pflanzen einsetzt, die ausgewählt sind aus der Gruppe, die gebildet wird von *Aesculus hippocastanum* (Rosskastanie), *Argania spinosa, Babtista tinctoria* (wilder Indigo), *Cantella asiatica, Camelilla sinensis* (Grüner Tee), *Chamonella recutita* (Kamille), *Ginkgo biloba* (Ginkgo), *Oleo europea* (Olive), *Litschi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna acontifolia,* und *Vitis vinifera* (wilder Wein) sowie deren Gemischen.

10. Verwendung nach mindestens einem der Ansprüche 1 bis 9, **dadurch gekennzeichnet, dass** man Mischungen einsetzt, die - bezogen auf die Wirkstoffe -
(a) 10 bis 90 Gew.-% Salicylsäure und/oder deren Derivate,
(b) 10 bis 90 Gew.-% Glyzyrrhetinsäure und/oder deren Derivate, und
(c) 0 bis 20 Gew.-% Pflanzenextrakte und/oder Vitamin B6
mit der Maßgabe enthalten, dass sich die Mengen zu 100 Gew.-% ergänzen.

11. Verwendung nach mindestens einem der Ansprüche 1 bis 10, **dadurch gekennzeichnet, dass** man die Wirkstoffmischungen in Form von Mikrokapseln einsetzt.

12. Verwendung nach mindestens einem der Ansprüche 1 bis 10, **dadurch gekennzeichnet, dass** man die Wirkstoffmischungen in Form von Pro-Liposomen einsetzt.

## Claims

1. Use of an active-component mixture containing
(a) salicylic acid and derivatives thereof and
(b) glycyrrhetic acid and derivatives thereof
for the production of a medicament for treating acne.

2. Use claimed in claim 1, **characterized in that** salicylic acid derivatives selected from the group consisting of alkali metal, alkaline earth metal, ammonium, alkylammonium, alkanolammonium, glucammonium and zinc salts of salicylic acid, esters of azelaic acid with linear or branched aliphatic C₁₋₁₈ alcohols and the full or partial esters of salicylic acid with polyols containing 2 to 15 carbon atoms and at least 2 hydroxyl groups are used as component (a).

3. Use claimed in claims 1 and/or 2, **characterized in that** salicylic acid or its zinc salt is used as component (a).

4. Use claimed in at least one of claims 1 to 3, **characterized in that** glycyrrhetic acid derivatives selected from the group consisting of alkali metal, alkaline earth metal, ammonium, alkylammonium, alkanolammonium, glucammonium and zinc salts of glycyrrhetic acid, esters of glycyrrhetic acid with linear or branched aliphatic C₁₋₁₈ alcohols and the full or partial esters of glycyrrhetic acid with polyols containing 2 to 15 carbon atoms and at least 2 hydroxyl groups are used as component (b).

5. Use claimed in at least one of claims 1 to 4, **characterized in that** an extract of *Glycyrrhiza glabra* is used as component (b).

6. Use claimed in at least one of claims 1 to 5, **characterized in that** the potassium, ammonium and/or zinc salt of glycyrrhetic acid or esters thereof with C₁₆₋₁₈ fatty alcohols are used as component (b).

7. Use as claimed in at least one of claims 1 to 6, **characterized in that** components (a) and (b) are used in a ratio by weight of 90:10 to 10:90.

8. Use claimed in at least one of claims 1 to 7, **characterized in that** plant extracts and/or vitamin B6 is/are used as an additional component (c).

9. Use as claimed in claim 8, **characterized in that** extracts of plants selected from the group consisting of *Aesculus hippocastanum* (horse chestnut), *Argania spinosa, Babtista tinctoria* (wild indigo), *Cantella asiatica, Camelilla sinensis* (green tea), *Chamonella recutita* (chamomile), *Gingko biloba* (gingko), *Oleo europa* (olive), *Litschi chinensis* (lychee), *Melissa officinalis* (lemon melissa), *Panax ginseng* (ginseng), *Passiflora incarnata* (passion flower), *Prunus dulcis* (sweet almond), *Pterocarpus marsupium, Ruscus aculeatus, Trifolium pratense* (red clover), *Uva ursi* (bearberry), *Vaccinium myrtillus* (blueberry), *Vigna acontifolia* and *Vitis vinifera* (wild vine) and mixtures thereof are used as component (c).

10. Use claimed in at least one of claims 1 to 9, **characterized in that** mixtures containing - based on the active components -
(a) 10 to 90% by weight salicylic acid and/or derivatives thereof,
(b) 10 to 90% by weight glycyrrhetic acid and/or derivatives thereof and
(c) 0 to 20% by weight plant extracts and/or vitamin B6,
with the proviso that the quantities add up to 100% by weight,
are used.

11. Use claimed in at least one of claims 1 to 10, **characterized in that** the active-component mixtures are used in the form of microcapsules.

12. Use claimed in at least one of claims 1 to 10, **characterized in that** the active-component mixtures are used in the form of pro-liposomes.

## Revendications

1. Utilisation d'un mélange de principes actifs contenant
(a) de l'acide salicylique ainsi que ses dérivés et
(b) de l'acide glycyrrhétinique ainsi que ses dérivés
pour la préparation d'un médicament contre l'acné.

2. Utilisation selon la revendication 1,
**caractérisé en ce que**
comme composant (a) on utilise des dérivés de l'acide salicylique choisis dans le groupe constitué de sels alcalins, alcalino-terreux, d'ammonium, d'alkylammonium, d'alcanolammonium, de glucammonium et de zinc de l'acide salicylique, des esters de l'acide azélaïque avec des alcools aliphatiques linéaires ou ramifiés ayant 1 à 18 atomes de carbone, ainsi que des esters entiers ou partiels de l'acide salicylique avec des polyols ayant 2 à 15 atomes de carbone et au moins 2 groupes hydroxyle.

3. Utilisation selon les revendications 1 et/ou 2,
**caractérisée en ce que**
comme composant (a) on utilise l'acide salicylique ou son sel de zinc.

4. Utilisation selon au moins l'une des revendications 1 à 3,
**caractérisé en ce qu'**
comme composant (b), on utilise des dérivés de l'acide glycyrrhétinique, choisis dans le groupe constitué de sels alcalins, alcalino-terreux, d'ammonium, d'alkylammonium, d'alcanolammonium, de glucammonium et de zinc de l'acide glycyrrhétinique, des esters de l'acide glycyrrhétinique avec des alcools aliphatiques linéaires ou ramifiés comportant 1 à 18 atomes de carbone, ainsi que des esters entiers ou partiels de l'acide glycyrrhétinique avec des polyols ayant 2 à 15 atomes de carbone et au moins 2 groupes hydroxyle.

5. Utilisation selon au moins l'une des revendications 1 à 4,
**caractérisée en ce que**
comme composant (b) on utilise un extrait de *Glycyrrhiza glabra.*

6. Utilisation selon au moins l'une des revendications 1 à 5,
**caractérisée en ce que**
comme composant (b), on utilise le sel de potassium, d'ammonium et/ou de zinc de l'acide glycyrrhétinique ou ses esters avec des alcools gras ayant 16 à 18 atomes de carbone.

7. Utilisation selon au moins l'une des revendications 1 à 6,
**caractérisée en ce qu'**
on utilise les composants (a) et (b) dans un rapport pondéral de 90/10 à 10/90.

8. Utilisation selon au moins l'une des revendications 1 à 7,
**caractérisée en ce que**
comme autre composant (c) on utilise des extraits végétaux et/ou de la vitamine B6.

9. Utilisation selon la revendication 8,
**caractérisée en ce que**
comme composant (c) on utilise des extraits de plantes choisies dans le groupe constitué d'*Aesculus hippocastanum* (marron d'inde), d'*Argania spinosa,* de *Babtista tinctoria* (indigo sauvage), de *Cantella asiatica,* de *Camelilla sinensis* (thé vert), de *Chamonella recutita* (camomille), de *Ginkgo biloba* (ginkgo), d'*Oelo europea* (olive), de *Litschi chinensis* (litchi), de *Melissa officinalis* (mélisse officinale), de *Panax ginseng* (ginseng), de *Passiflora incarnata* (passiflore), de *Prunus dulcis* (amande douce), de *Pterocarpus marsupium,* de *Ruscus aculeatus,* de *Trifolium pratense* (trèfle violet), d'*Uva ursi* (busserolle), de *Vaccinium myrtillus* (myrtille), de *Vigna acontifolia* et de *Vitis vinifera* (vigne vierge) ainsi que leurs mélanges.

10. Utilisation selon au moins l'une des revendications 1 à 9,
**caractérisé en ce qu'**
on utilise des mélanges qui contiennent - par rapport aux principes actifs -
(a) 10 à 90 % d'acide salicylique et/ou ses dérivés,
(b) 10 à 90 % d'acide glycyrrhétinique et/ou ses dérivés, et
(c) 0 à 20 % en poids d'extraits végétaux et/ou de vitamine B6,
étant précisé que les quantités se complètent à 100 % en poids.

11. Utilisation selon au moins l'une des revendications 1 à 10,
**caractérisée en ce qu'**
on utilise les mélanges de principes actifs sous forme de microcapsules.

12. Utilisation selon au moins l'une des revendications 1 à 10,
**caractérisée en ce qu'**
on utilise les mélanges de principes actifs sous forme de pro-liposomes.
